(19) Europäisches Patentamt
European Patent Office
Office européen des brevets

(11) Veröffentlichungsnummer: **0 130 327**
**B1**

(12) # EUROPÄISCHE PATENTSCHRIFT

(45) Veröffentlichungstag der Patentschrift: **04.11.87**

(51) Int. Cl.⁴: **C 07 H 13/12,** A 61 K 31/70

(21) Anmeldenummer: **84105508.0**

(22) Anmeldetag: **15.05.84**

(54) **Moenomycin A-Derivate, Verfahren zu ihrer Herstellung und ihre Verwendung als Antibiotika.**

(30) Priorität: **21.05.83 DE 3318594**

(43) Veröffentlichungstag der Anmeldung: **09.01.85 Patentblatt 85/2**

(45) Bekanntmachung des Hinweises auf die Patenterteilung: **04.11.87 Patentblatt 87/45**

(84) Benannte Vertragsstaaten: **CH DE FR GB IT LI**

(56) Entgegenhaltungen:
**DE-A-3 221 732**

**TETRAHEDRON, Band 39, Nr. 9, 1983, Seiten 1583-1591, Pergamon Press Ltd., GB; P.Welzel et al.: "Moenomycin AL: further structural studies and preparation of simple derivatives"**
**CARBOHYDRATE RESEARCH, Band 126, 1984, Seiten C1-C5, Elsevier Science Publishers B.V., Amsterdam, NL; P.Welzel et al.: "Stepwise degradation of moenomycin A".**

(73) Patentinhaber: **HOECHST AKTIENGESELLSCHAFT, Postfach 80 03 20, D-6230 Frankfurt am Main 80 (DE)**

(72) Erfinder: **Welzel, Peter, Prof. Dr., Hustadtring 81, D-4630 Bochum (DE)**
Erfinder: **Kunisch, Franz, Alsenstrasse 10, D-4630 Bochum (DE)**
Erfinder: **Stein, Hermann, Dr., Hochstrasse 33, D-4390 Gladbeck (DE)**
Erfinder: **Hiltmann geb. Ponty, Aranka, Wehlaustrasse 46, D-4630 Bochum (DE)**
Erfinder: **Kruggel, Frithjof, Gustavstrasse 16, D-4630 Bochum (DE)**

**EP 0 130 327 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Aus Moenomycin A der Struktur 1 (Angewandte Chemie 93, 130, 1981), wird gemäß US 3 432 597 durch katalytische Hydrierung Dekahydromoenomycin A der Formel 2 erhalten, das nach der DE-A-32 21 732 durch Ozonisierung unter Abspaltung des Chromophorbausteins A in die antibiotisch aktive Verbindung 3 umgewandelt wird, die noch die im Moenomycin ursprünglich enthaltenen Zuckerbausteine D-Galacturonsäure (B), N-Acetyl-D-Glucosamin (C), D-Glucose (D), N-Acetyl-D-Chinovosamin (E) und Moenuronsäure (F) enthält.

In der DE-A-33 01 430 wurde beschrieben, daß auch die sukzessive Abspaltung der Zuckerbausteine D und B von der Verbindung 3 jeweils zu neuen antibiotisch aktiven Verbindungen (7 und 8) führt. Zur Abspaltung des Zuckerbausteins D-Glucose (d) aus 3 wird zunächst durch Umsetzung von 3 mit Benzaldehyd in Gegenwart von wasserfreiem Zinkchlorid das Dibenzyliden-Derivat 5 und daraus anschliegend mit $NaJO_4$ in Essigsäure unter Abspaltung von D das Monobenzyliden-Derivate 6 hergestellt, das weiterhin durch katalytische Hydrierung, zweckmäßig mit Palladiumkohle, unter Abspaltung des Benzylrestes in die glucosefreie Verbindung 7 überführt wird. Die anschließendc Abspaltung des Zuckerbausteins D-Galacturonsäure (B) erfolgt in analoger Weise aus 7 in Essigsäure, wobei 8 erhalten wird.

Die genannten deutschen Offenlegungsschriften wurden nach dem Prioritätstag der vorliegenden Erfindung veröffentlicht.

Es wurde nun überraschenderweise gefunden, daß auch die weitere Abspaltung des Zuckerbausteins N-Acetyl-D-Chinovosamin (C) aus 8 mit $NaJO_4$ in Essigsäure unter Bildung von 9 sowie die anschließende Abspaltung des Zuckerbausteins N-Acetyl-D-Glukosamin (E) unter Bildung von 10 jeweils zu neuen antibiotisch aktiven Verbindungen (9 und 10) führt.

Gegenstand der Erfindung ist daher eine Verbindung der Formel 9

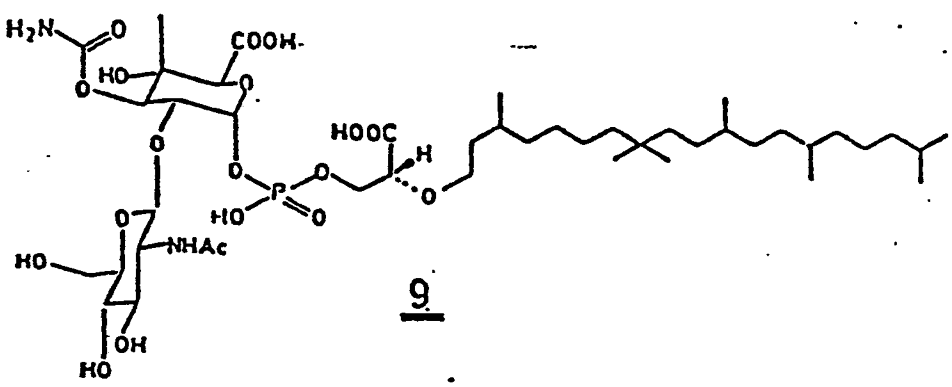

9

und eine Verbindung der Formel 10

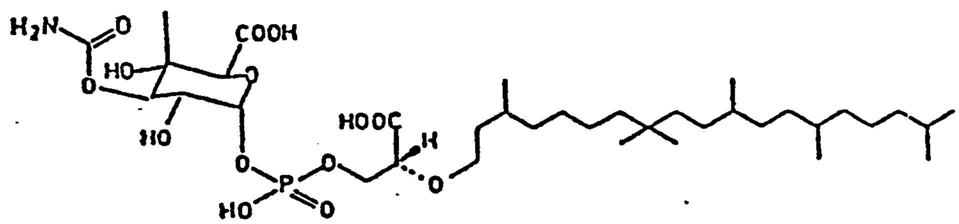

10

Gegenstand der Erfindung ist ebenso ein Verfahren zur Herstellung der Verbindung der Formel 9, dadurch gekennzeichnet, daß man eine Verbindung der Formel 8

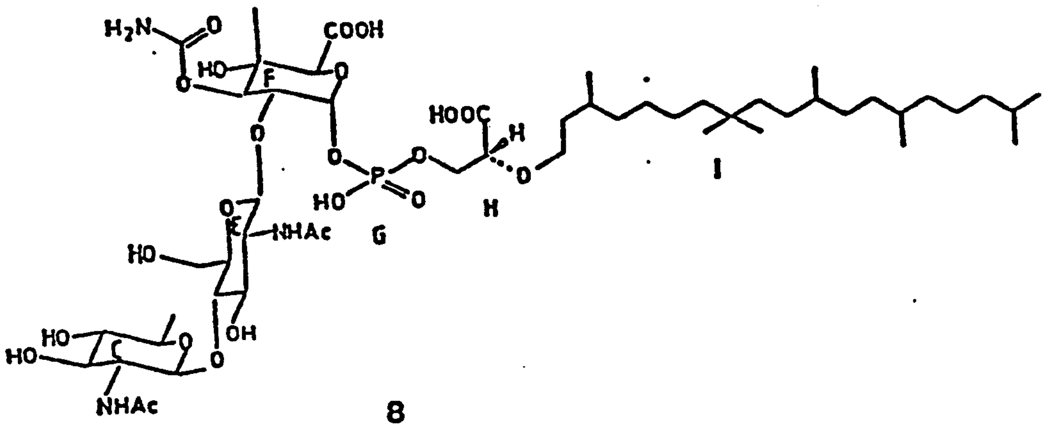

8

einer $NaJO_4$-Spaltung unterwirft.

Gegenstand der Erfindung ist ebenso ein Verfahren zur Herstellung der Verbindung 10 dadurch gekennzeichnet, daß man eine Verbindung der Formel 9 einer $NaJO_4$-Spaltung unterwirft.

Gegenstand der Erfindung sind weiterhin die Verwendung der Verbindung der Formel 9 als Antibiotikum und die Verwendung der Verbindung der Formel 10 als Antibiotikum.

Die Reaktionsfolgen werden durch folgendes Formelschema erläutert:

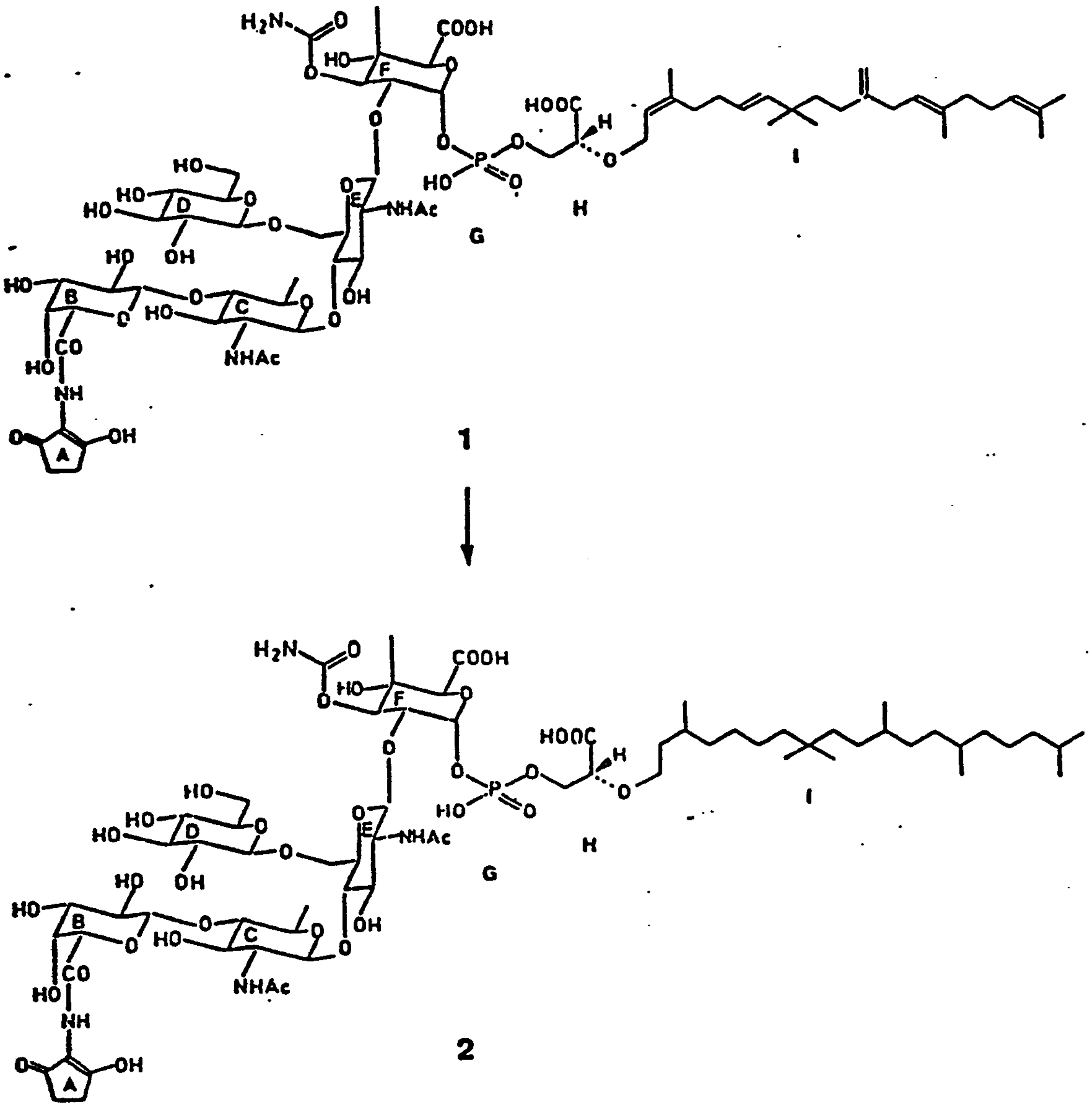

1

2

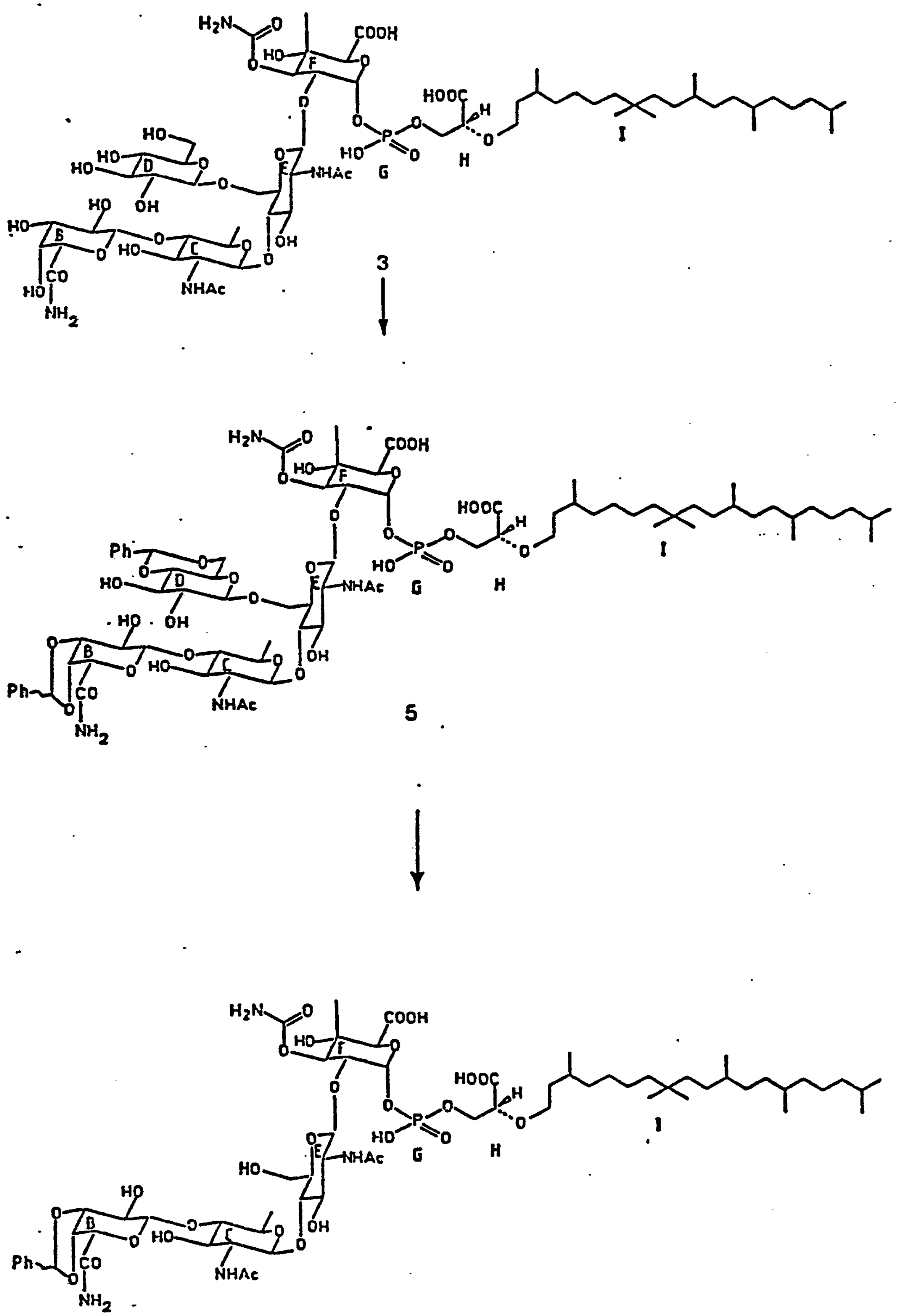

3

5

6

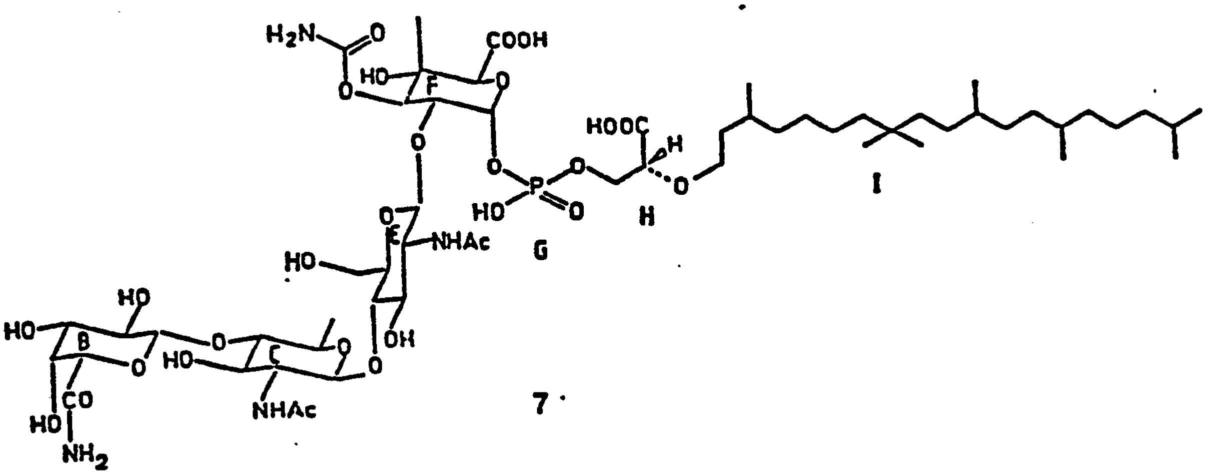

7

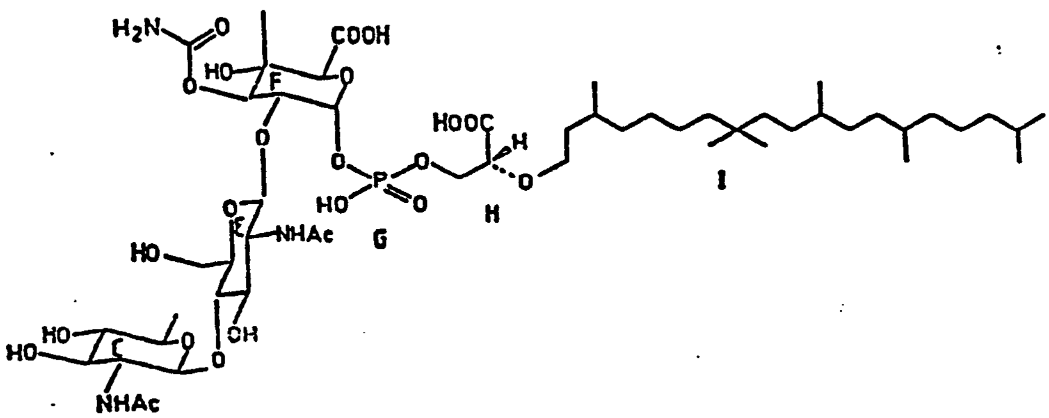

8

$NaJO_4$

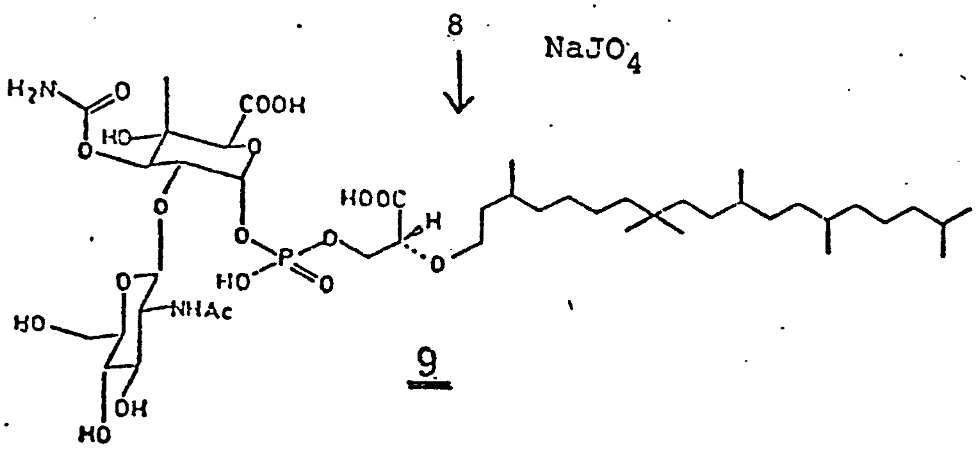

9

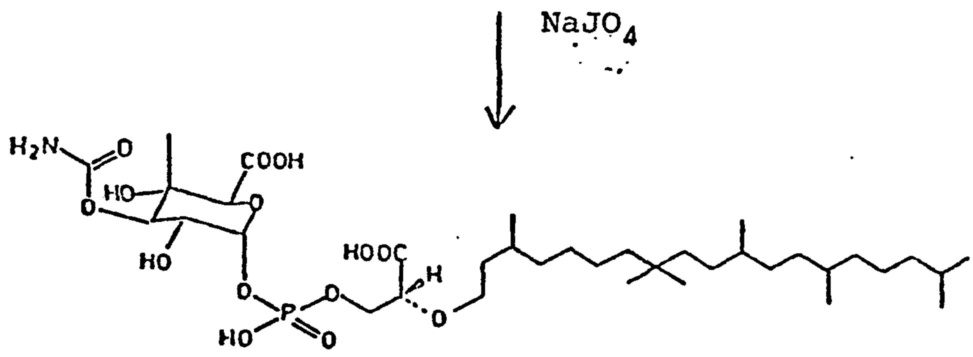


10

Die bereits in der DE-A-33 01 430 beschriebenen Verbindungen 7 und 8 sowie die erfindungsgemäßen Verbindungen 9 und 10 besitzen gegenüber Moenomycin A verbesserte Stabilität und sind antibiotisch aktiv, wie sich aus folgender Tabelle ergibt:

Tabelle 1: Antimikrobielle Aktivität (minimale Hemmkonzentration in µg/ml)

| Testkeim | | 7 | 8 | 9 | 10 |
|---|---|---|---|---|---|
| Staph. aureus SG | 511 | 3,1 | 6,2 | 12,5 | - |
| " | 503 | 3,1 | 12,5 | 12,5 | - |
| Strept. pyogenes | 77 A | 0,8 | 1,6 | 1,6 | 3,1 |

Die in der Beschreibung erwähnten Stufen werden durch folgende Beispiele erläutert:

**Beispiel 1**

Variante a)

14,6 g Moenomycin A (1) und 4,5 g Pd/C (10 % Pd; 4,2 mmol) wurden in 350 ml Methanol bei 45°C unter Wasserstoff 180 bar) 96 h gerührt. Nach Abdekantieren vom Katalysator wurde dreimal mit Methanol gewaschen und nach Vereinigen der Lösungen bei 35°C im Vakuum zur Trockne eingeengt. Ausbeute an Dekahydromoenomycin (2) 14 g (96 %).

Variante b)

13,09 g Moenomycin A (1, verunreinigt mit Deslipidomoenomycin) und 3,53 g $PtO_2$ wurden in 1,3 l Methanol unter Zugabe von 43 ml Essigsäure bei Raumtemperatur und Normaldruck unter Wasserstoff 48 h gerührt. Nach Abdekantieren vom Katalysator wurde viermal mit Methanol gewaschen und auf 220 g HP-20 aufgegeben. Die Elution erfolgte mit 2 l Wasser, 1 l Methanol/Wasser 25/75, 1 l Methanol/Wasser 50/50 und 3 l Methanol. Die Methanol-Fraktion wurde zur Trockne eingeengt und lieferte 8,04 g (61 %) Dekahydromoenomycin (2).

Zu 11,67 g (7,4 mmol) 2 in 300 ml Wasser wurden unter Argon 26,6 g (80,8 mmol) $K_3Fe(CN)_6$ in 30 ml Wasser und 16,72 g (121 mmol) $K_2CO_3$ in 30 ml Wasser unter Rühren bei 0°C zugegeben. Innerhalb von 0,5 h wurde auf Raumtemperatur erwärmt und weitere 1,5 h gerührt. Die Salze wurden durch reversed-phase-Chromatographie (220 g HP-20, Laufmittel: 1,5 l Wasser und 2,2 l Methanol) entfernt. Einengen der Methanol-Lösung ergab 9,7 g 3 (88 %).

**Beispiel 3**

Zu einer Lösung von 1,34 g (9,9 mmol) wasserfreiem Zinkchlorid in 3 ml (25 mmol) Benzaldehyd wurden 1,06 g (0,72 mmol) 3, gelöst in 3 ml trockenem DMSO, bei 90°C unter Argon zugegeben und 52 h bei 90-95°C gerührt. Die dunkelbraune Reaktionslösung wurde durch reversed-phase-Chromatographie (70 g HP-20, Laufmittel 200 ml Wasser, 150 ml Methanol/Wasser 1:1, 200 ml Methanol/Wasser 6:4, 100 ml Methanol/Wasser 7:3 und ca. 1000 ml Methanol) vorgereinigt.

1416 mg des lyophilisierten, dunkelgelben Methanol-Eluats wurden auf 5 g Kieselgel aufgezogen und mit dem Laufmittel Chloroform/Methanol/Wasser 10:6:1 an 280 g Kieselgel "Merck" (Säule Typ C) getrennt (Fraktionsvolumen ca. 12 ml).

Die 5 enthaltenden Fraktionen 37-66 wurden erneut unter den gleichen Bedingungen getrennt. Die Gesamtausbeute an 5 betrug 92 mg (8 %).

**Beispiel 4**

200 mg (0,12 mmol) 5 wurden innerhalb von 45 min zu 1200 µl einer Lösung von 200 mg (0,93 mmol) $NaJO_4$, 260 mg Natriumacetat · 3 $H_2O$ und ca. 2,4 ml 50 % Essigsäure zugegeben und 5 h bei 38-40°C unter Lichtausschluß gerührt. Während der Reaktion bildete sich ein orangefarbener Niederschlag. Zur Abtrennung der Salze wurde die Reaktionsmischung auf eine HP-20 reversed-phase Säule aufgetragen und zuerst mit 70 ml Wasser, dann mit ca. 400 ml Methanol eluiert. Nach Lyophilisieren wurden 170 mg (85 %) eines unpolareren Produktes ($R_f$-Wert 0,33 für Chloroform/Methanol/Wasser 18:11:2,7) erhalten.

95 mg (0,063 mmol) dieses Oxidationsproduktes wurden zu 240 µl einer Lösung aus 470 µl (6,2 mmol) N,N-Dimethylhydrazin, 1,4 ml Isopropylalkohol und ca. 2,8 ml 2n $H_2SO_4$ (pH-Wert der Lösung: 4,0) zugegeben und 2,5 g bei Raumtemperatur und 1 h bei 80 - 90°C gerührt. Der Reaktionsansatz wurde mit 5 ml Wasser versetzt und lyophilisiert. Nach Aufziehen auf ca. 0,5 g Kieselgel "Merck" wurde mit dem Laufmittel Chloroform/Methanol/Wasser 10:6:1 an 60 g Kieselgel (Säule Typ B) getrennt. Es wurden 35 mg (43 %) 6 erhalten ($R_f$-Wert 0,3 für Chloroform/Methanol/Wasser 18:11:2,7).

**Beispiel 5**

180 mg (0,126 mmol) 6 wurden in 0,5 ml Methanol und 4 ml Essigsäure gelöst und bei Raumtemperatur nach Zugabe von 200 mg Pd/C (10 %) 44 h unter Wasserstoff hydriert. Die Reaktion verlief dünnschichtchromatographisch (Chloroform/Methanol/Wasser 18:11:2,7) nahezu einheitlich. Nach mehrmaligem Waschen des Katalysators mit Methanol wurde eingeengt und lyophilisiert. Es wurden 186,4 mg (100 %) des Produktes 7 ($R_f$-Wert 0,1 für Chloroform/Methanol/Wasser 18:11:2,7) erhalten.

**Beispiel 6**

Zu 165 mg (0,12 mmol) in 0,6 ml Wasser gelöstem 7 wurden 300 µl einer Lösung bestehend aus 200 mg (0,93 mmol) $NaJO_4$, 260 mg Natriumacetat · 3 $H_2O$ und 2,4 ml 50 % Essigsäure zugegeben und bei 25°C 2 h gerührt. Nach 2 h, 6 h, 19 h, 21 h, 25 h und 30 h wurden jeweils 150 µl, 190 µl, 150 µl, 100 µl, 200 µl und 300 µl der $NaJO_4$ enthaltenden Lösung zugegeben und die Temperatur nach 10,5 h auf 30°C und nach 25 h auf 40°C erhöht. Nach 44 h Reaktionsdauer wurden Natriummetaperjodat und Natriumacetat über eine HP-20 reversed-phase Säule abgetrennt. Die Elution mit ca. 500 ml Methanol ergab nach Einengen 122 mg (74 %) Produktgemisch.

Zu der Suspension von 122 mg dieses Produktgemisches in 700 µl Isopropylalkohol wurden 260 µl einer Lösung bestehend aus 470 µl N,N-Dimethylhydrazin, 1,4 ml Isopropylalkohol und 2,5 ml 2n $H_2SO_4$ (pH-Wert der Lösung: 4,0 - 4,5) zugegeben und 1 h bei 50°C und 1 h bei 80 - 85°C gerührt. Nach Aufziehen auf 1 g Kieselgel wurde ein 60 g Kieselgel "Merck" (Säule, Typ B) mit dem Laufmittel Chloroform/Methanol/Wasser 18:11:2,7 getrennt und 66 mg (45 %) der Substanz 8 ($R_f$ -Wert 0,21 für Chloroform/Methanol/Wasser 18:11:2,7) erhalten.

**Beispiel 7**

1,0 g (0,7 mmol) 3 wurde portionsweise fest zu einer Lösung von 1,0 g (4,7 mmol) $NaJO_4$, 1,3 g Natriumacetat · 3 $H_2O$ in 12 ml 50 % Essigsäure zugesetzt. Unter Lichtausschluß wurde 2 h bei 40°C gerührt. Anschließend wurde der bräunlich gefärbte Reaktionseinsatz durch reversed-phase-Chromatographie (40 g HP-20; Laufmittel: 600 ml Wasser und 600 ml Methanol) gereinigt. Nach Einengung und Lyophilisieren wurden aus der Methanollösung 830 mg Oxidationsprodukte erhalten. 687 mg dieses Gemisches wurden portionsweise zu 3 ml einer Lösung von 940 µl N,N-Dimethylhydrazin und 2,8 ml 2-Propanol in 6,4 ml 2n $H_2SO_4$ (pH: 4,5) zugegeben. Es wurde 3 h bei 85°C gerührt. Nach Abkühlen wurde durch reversed-phase-Chromatographie (40 g HP-20; Laufmittel: 600 ml Wasser, 600 ml Methanol) gereinigt. Die Methanollösung wurde eingedampft und lyophilisiert. Zweimalige Säulenchromatographie an Kieselgel "Merck" (Säule B, Laufmittel: Chloroform/Methanol/Wasser 10:6:1) ergab 311 mg (47 %) 8.

**Beispiel 8**

2,35 g (2,0 mmol) 8 wurde portionsweise fest zu einer Lösung von 1,84 g (8,6 mmol) $NaJO_4$, 2,41 g (17,7 mmol) $NaOAc \cdot 3\ H_2O$ in 21 ml 50 % Essigsäure zugesetzt. Unter Lichtausschluß wurde zwei Stunden bei Raumtemperatur gerührt. Durch reversed-phase-Chromatographie (50 g HP-20; erst 700 ml Wasser, dann 1,5 l Methanol) wurden die anorganischen Salze abgetrennt. Einengen und Lyophilisieren der Methanollösung lieferte 1,76 g hellgelbes Oxidationsprodukt.

1,76 g dieser Substanz wurden portionsweise zu 6 ml einer Lösung von 940 µl N,N-Dimethylhydrazin und 2,8 ml 2-Propanol in 6,4 ml 2n $H_2SO_4$ (pH 4,5) gegeben. Man rührte 3 h bei 80 - 85°C und trug nach Abkühlen die dunkelbraune Lösung auf eine HP-20-Säule auf. Man eluierte die Salze mit 600 ml Wasser, die Produkte mit 1,2 l Methanol. Einengen und Lyophilisieren dieser Phase ergab 1,48 g Rohprodukt.

Präparative Schichtchromatographie an 75 g Kieselgel (Woelm 63 - 100 µ, Laufmittel Chloroform/Methanol/Wasser 10 : 6 : 1) lieferte 1,02 g (52 %) 9.

Zwei Nachtrennungen an Kieselgel-Säulen mit demselben Laufmittel erbrachte aus 210 mg Produkt 69,8 mg analytisch reine Substanz.

$^{13}$C-NMR-Spektrum: Figur 1

**Beispiel 9**

940 mg (0,96 mmol) 9 wurden zwei Stunden im Dunkeln bei Raumtemperatur mit einer Lösung aus 974 mg (4,6 mmol) $NaJO_4$, 1,28 g $NaOAc \cdot 3\ H_2O$ in 11 ml 50 % Essigsäure gerührt. Aufarbeitung über eine reversed-phase-Säule (25 g HP-20, Laufmittel 400 ml Wasser, dann 1,5 l Methanol) ergab nach Einengen und Lyophilisieren der Methanollösung 648 mg gelbe Substanz.

620 mg dieses Produkts wurden mit 4 ml einer Lösung aus 940 µl N,N-Dimethylhydrazin und 2,8 ml 2-Propanol in 6,4 ml 2n $H_2SO_4$ (pH 4,5) erst eine Stunde bei Raumtemperatur, dann zwei Stunden bei 80 - 85°C gerührt. Nach Abkühlen und Auftragen auf eine HP-20-Säule (25 g) wurden die Salze mit 300 ml Wasser, die Produkte mit 1,5 l Methanol eluiert. Aus der Methanolphase wurden nach Lyophilisieren 0,54 g hellbraunes Produkt erhalten.

Präparative Schichtchromatographie an Kieselgel (Merck B-Säule, Laufmittel Chloroform/Methanol/Wasser 10 : 6 : 1) ergab 138 mg 10 (19 %) neben 296 mg 9 (31 %).

Zwei Nachtrennungen über B-Säulen mit dem Laufmittel Chloroform/Methanol/Wasser 17,5 : 7,5 : 1 erbrachten aus 138 mg 10 60,3 mg analytisch reine Substanz.

$^{13}$C-NMR-Spektrum: Figur 2

**Patentansprüche**

1. Verbindung der Formel 9

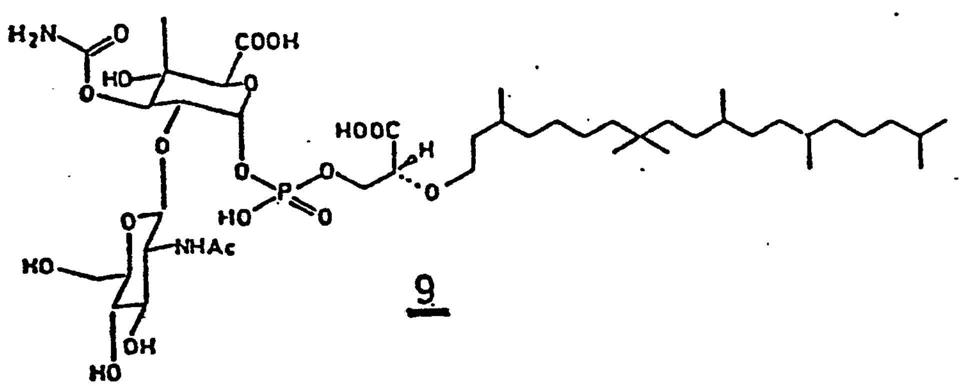

9

2. Verbindung der Formel, 10

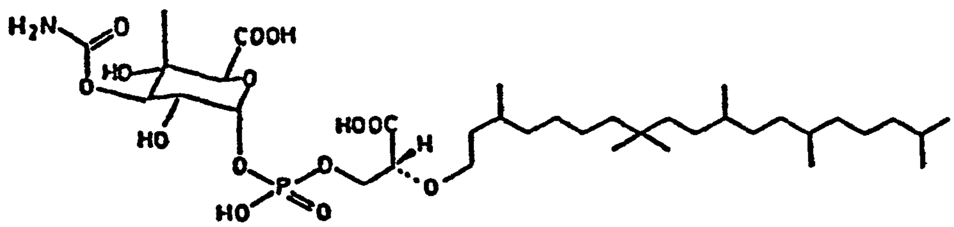

10

3. Verfahren zur Herstellung der Verbindung der Formel 9 nach Anspruch 1, dadurch gekennzeichnet, daß man eine Verbindung der Formel 8

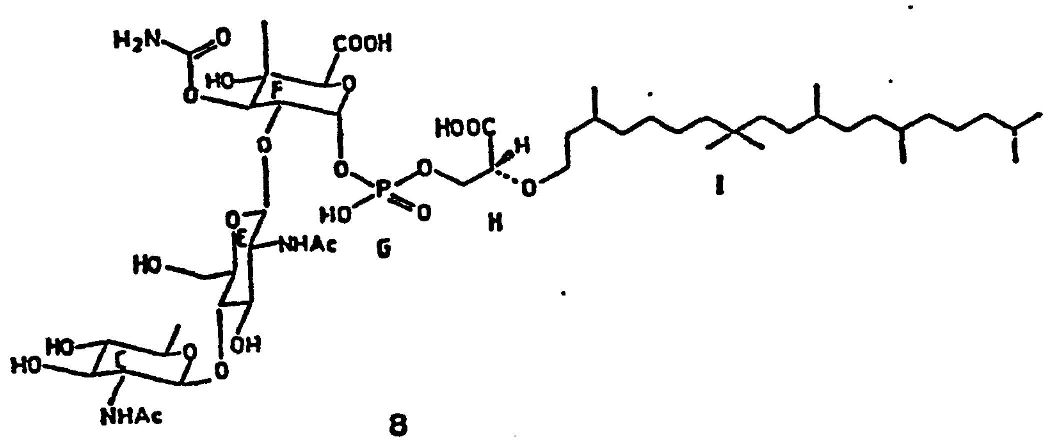

8

einer $NaJO_4$-Spaltung unterwirft.

4. Verfahren zur Herstellung der Verbindung 10 nach Anspruch 2, dadurch gekennzeichnet, daß man eine Verbindung der Formel 9 nach Anspruch 1 einer $NaJO_4$-Spaltung unterwirft.

5. Verbindung der Formel 9 nach Anspruch 1 zur Verwendung als Antibiotikum.

6. Verbindung der Formel 10 nach Anspruch 2 zur Verwendung als Antibiotikum.

**Claims**

1. A compound of the formula 9

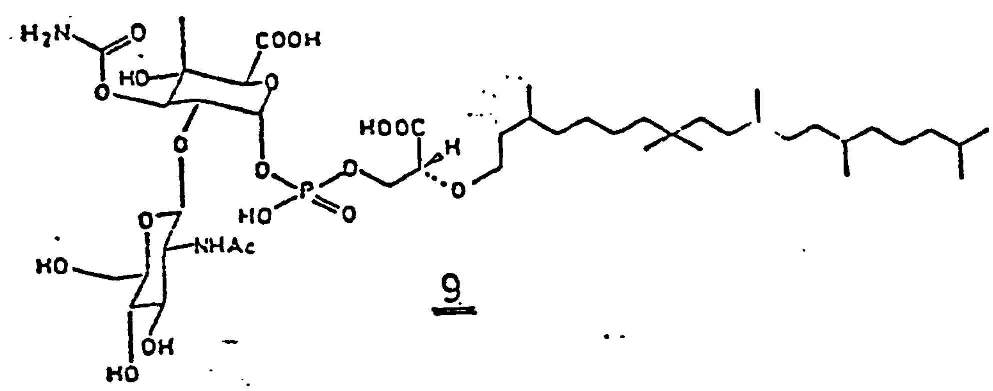

9

2. A compound of the formula 10

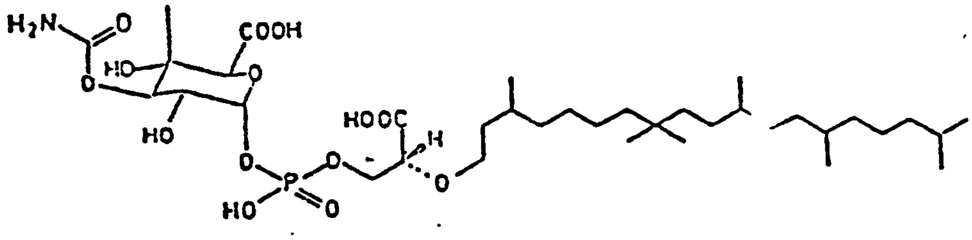

10

3. A process for the preparation of the compound of the formula 9 as claimed in Claim 1, which comprises subjecting a compound of the formula 8

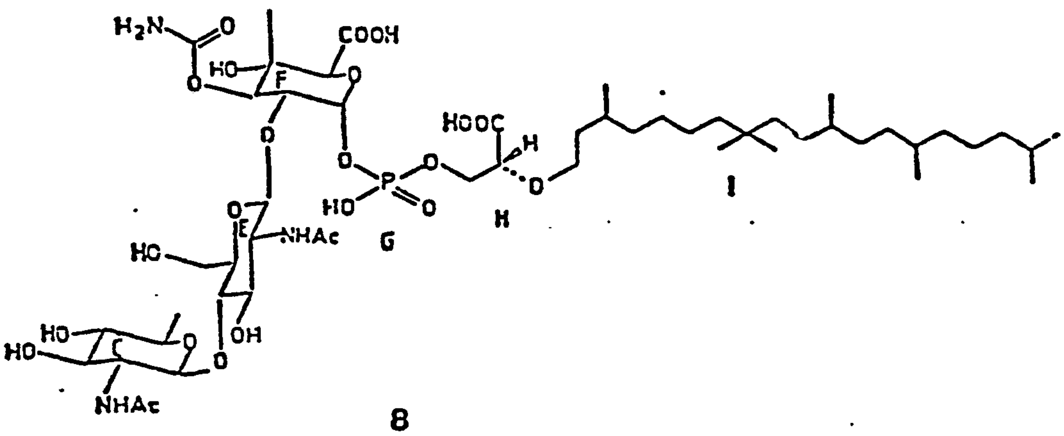

8

to cleavage with $NaIO_4$.

4. A process for the preparation of the compound 10 as claimed in Claim 2, which comprises subjecting a compound of the formula 9, as claimed in Claim 1, to cleavage with $NaIO_4$.

5. Compound of the formula 9, as claimed in Claim 1, for use as an antibiotic.

6. Compound of the formula 10, as claimed in Claim 2, for use as an antibiotic.

**Revendications**

1. Composé de formule 9

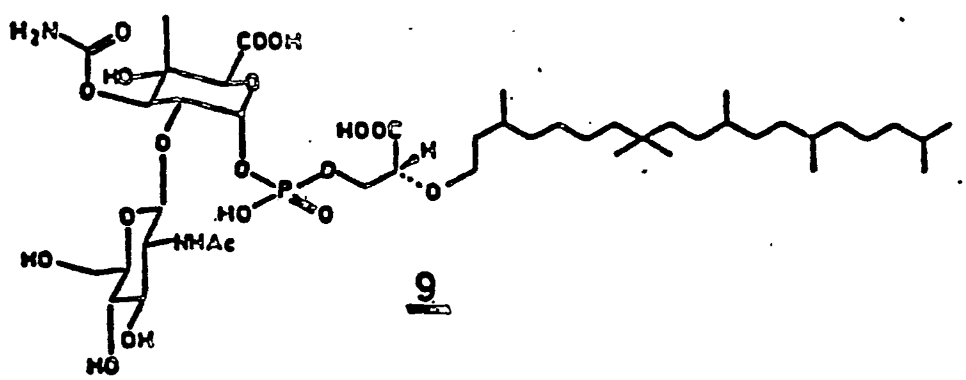

9

2. Composé de formule 10

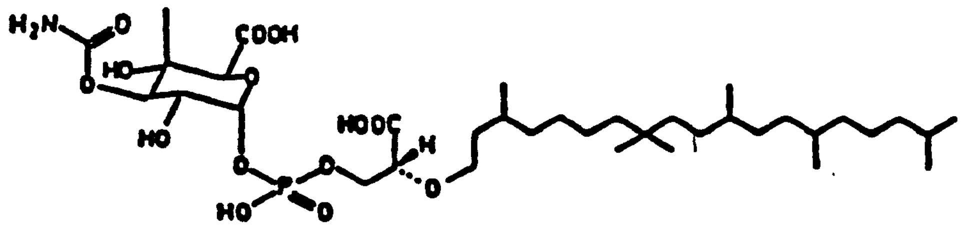

10

3. Procédé pour la préparation du composé de formule 9 selon la revendication 1, caractérisé en ce qu'on soumet un composé de formule 8

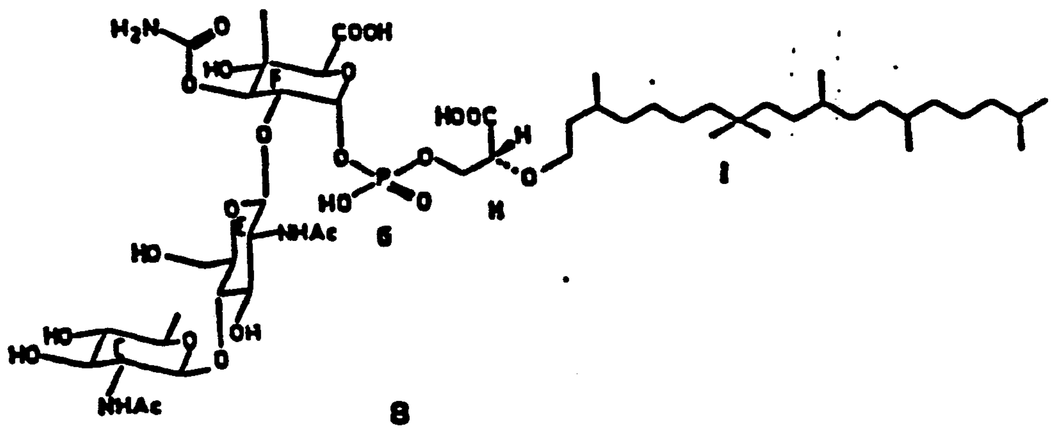

8

à une coupure avec $NaIO_4$.

4. Procédé pour la préparation du composé 10 selon la revendication 2, caractérisé en ce qu'on soumet un composé de formule 9 selon la revendication 1 à une coupure avec $NaIO_4$.

5. Composé de formule 9 selon la revendication 1, pour utilisation comme antibiotique.

6. Composé de formule 10 selon la revendication 2, pour utilisation comme antibiotique.

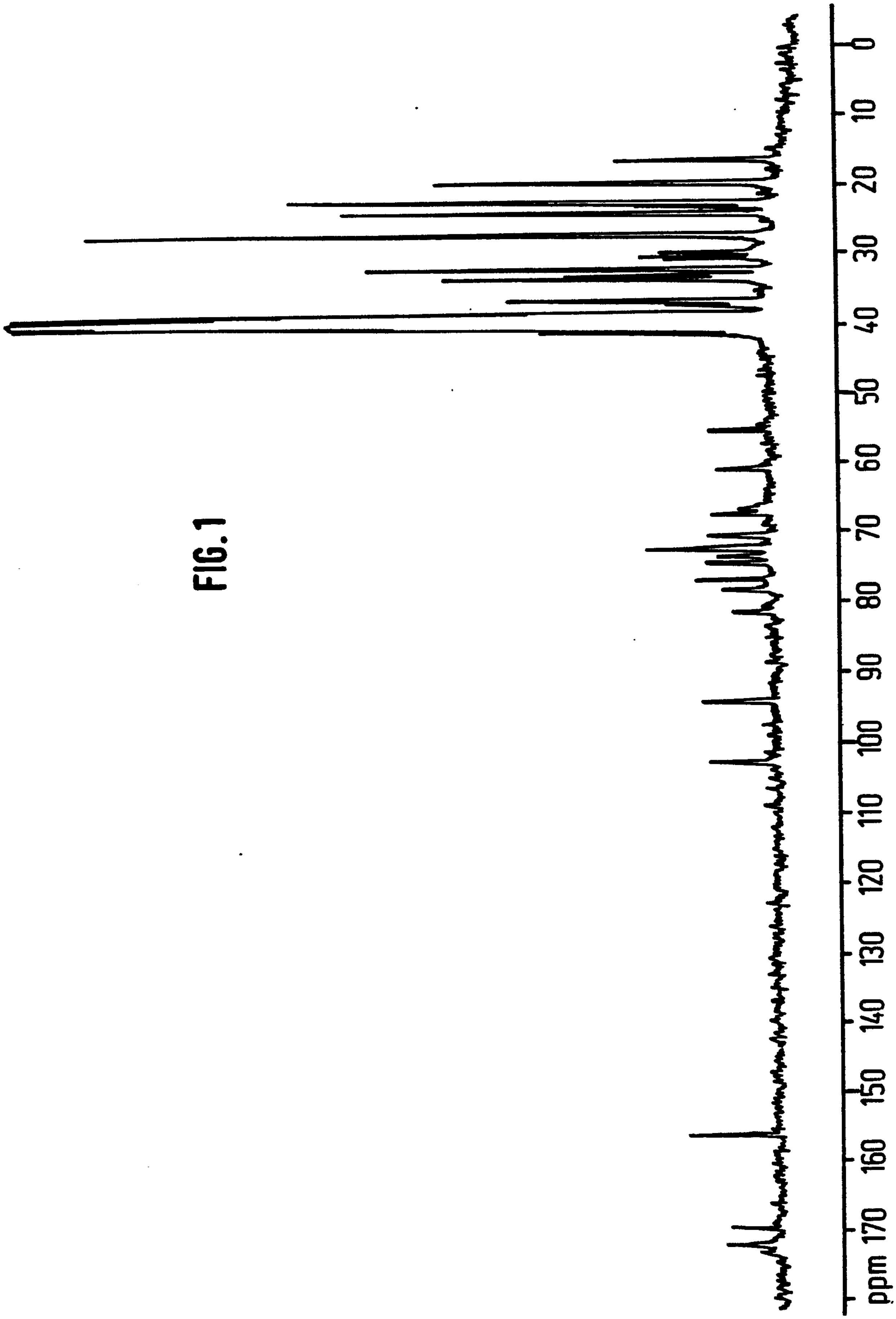


FIG. 1

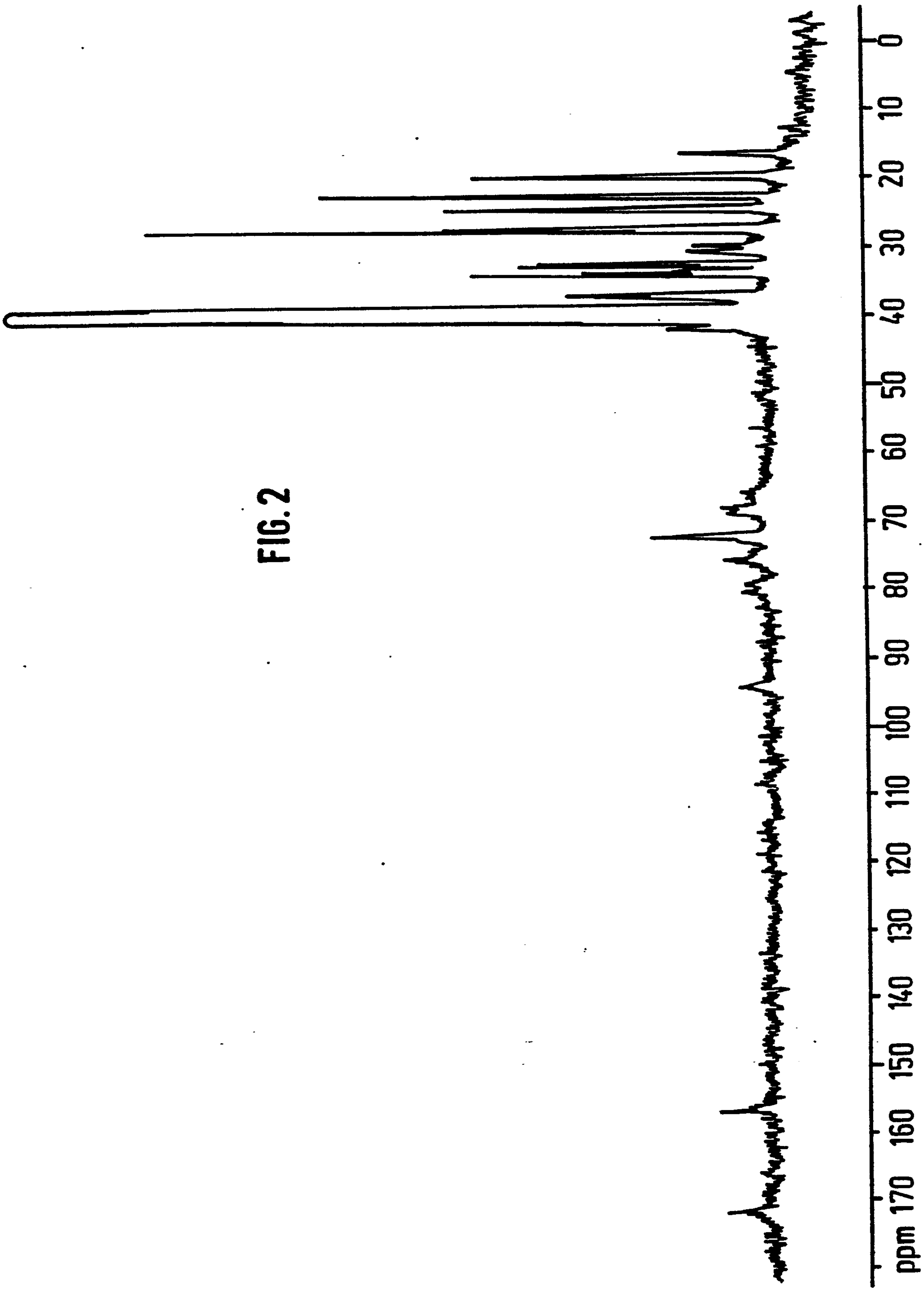


FIG. 2